# EUROPEAN PATENT APPLICATION

(11) **EP 2 829 966 A1**
(43) Date of publication of application: **28.01.2015**
(21) Application number: 13194991.9
(22) Date of filing: 29.11.2013
(51) Int. Cl.: G06F 3/0488, A61B 8/00, G06F 1/16, G06F 19/00

(54) **Hardware device, user control apparatus for the same, medical apparatus including the same, and method of operating medical apparatus**

(30) Priority: 24.07.2013 KR 20130087610
(71) Applicant: Samsung Medison Co., Ltd., Gangwon-do 250-870 (KR)
(72) Inventor: Jin, Gil-ju, Gangwon-do (KR); Ahn, Mi-jeoung, 250-870 Gangwon-do (KR); Hyun, Dong-gyu, Gangwon-do (KR)
(74) Representative: Schmid, Wolfgang

(57) **Abstract**

Provided are a hardware device, a user control apparatus for the same, a medical apparatus including the same, and a method of operating the medical apparatus. The method includes sensing a pattern of a hardware device disposed on a touch screen, and when the sensed pattern matches a stored pattern, determining the hardware device as an input apparatus enabling a user command to be input.

## Description

### RELATED APPLICATIONS

This application claims the benefit of Korean Patent Application No. 10-2013-0087610, filed on July 24, 2013, in the Korean Intellectual Property Office, the disclosure of which is incorporated herein in its entirety by reference.

### BACKGROUND

### 1. Field

One or more embodiments of the present invention relate to a hardware device, a user control apparatus for the same, a medical apparatus including the same, and a method of operating the medical apparatus.

### 2. Description of the Related Art

Ultrasonic apparatuses, magnetic resonance imaging (MRI) apparatuses, computed tomography (CT) apparatuses, and X-ray apparatuses are widely used as medical apparatuses for acquiring a medical image of a human body. Such apparatuses may capture a part or all of a human body, depending on, for example, a resolution of an image or a size of an apparatus itself. Also, when capturing all of a human body, the medical apparatuses may capture the human body at one time, or may capture parts of the human body several times and synthesize the captured images to acquire a synthesized image of all of the human body.

A user control apparatus for the medical apparatuses may be implemented as a touch screen. However, when the user control apparatus is implemented as the touch screen, the user control apparatus causes inconvenience to users skilled in relation to hardware keys that give the users a haptic sense.

### SUMMARY

One or more embodiments of the present invention include a hardware device, a user control apparatus for the same, a medical apparatus including the same, and a method of operating the medical apparatus.

Additional aspects will be set forth in part in the description which follows and, in part, will be apparent from the description, or may be learned by practice of the presented embodiments.

According to one or more embodiments of the present invention, a method of operating a medical apparatus includes: sensing a pattern of a hardware device disposed on a touch screen; and determining the hardware device as an input apparatus enabling a user command to be input when the sensed pattern matches a stored pattern.

The pattern of the hardware device may be formed at a surface of the hardware device facing the touch screen.

The method may further include controlling the medical apparatus according to a motion of the hardware device.

The motion of the hardware device may correspond to a touch that is sensed in an area of the touch screen in which the hardware device is disposed.

The motion of the hardware device may be a motion of a partial area of the hardware device when a housing for the hardware device is fixed.

The method may further include displaying an object of a user interface associated with the hardware device, in an area near an area of the touch screen in which the hardware device is disposed.

When a position of the hardware device disposed on the touch screen is changed, a position of the object of the user interface may changed, and the changed object may be displayed.

The hardware device may include at least one of a trackball, a knob, a button, a slide bar, and a keyboard.

The medical apparatus may be a disposable apparatus.

According to one or more embodiments of the present invention, a medical apparatus includes: a touch screen that senses a pattern of a hardware device; a storage unit that stores a pattern and an object of a user interface, the pattern being mapped to the object of the user interface; and a control unit that, when a pattern of a hardware device disposed on the touch screen is included in the pattern stored in the storage unit, determines the hardware device as an input apparatus enabling a user command to be input.

The pattern of the hardware device may be formed at a surface of the hardware device facing the touch screen.

The control unit may control the medical apparatus according to a motion of the hardware device.

The motion of the hardware device may correspond to a touch that is sensed in an area of the touch screen in which the hardware device is disposed.

The motion of the hardware device may be a motion of a partial area of the hardware device when a housing for the hardware device is fixed.

The control unit may further display an object of a user interface associated with the hardware device, in an area near an area of the touch screen in which the hardware device is disposed.

When a position of the hardware device disposed on the touch screen is changed, the control unit may change a position of the object of the user interface and display the changed object.

The hardware device may include at least one of a trackball, a knob, a button, a slide bar, and a keyboard.

According to one or more embodiments of the present invention, a user control apparatus include: a touch screen that senses a pattern of a hardware device; and a control unit that, when a pattern of a hardware device disposed on the touch screen is included in the pattern stored in the storage unit, determines the hardware device as an input apparatus enabling a user command to be input.

According to one or more embodiments of the present invention, The touch screen may sense a motion of a partial area of the hardware device when the hardware is disposed on the touch screen.

According to one or more embodiments of the present invention, a hardware device for a user control apparatus includes: an identification unit that identifies the hardware device, disposed at a surface facing a touch screen that is an external device, as an object of a user interface; an operation unit that transfers a user command to the touch screen by using a motion; and a housing that forms an external appearance of the hardware device, and supports the identification unit and the operation unit.

### BRIEF DESCRIPTION OF THE DRAWINGS

These and/or other aspects will become apparent and more readily appreciated from the following description of the embodiments, taken in conjunction with the accompanying drawings of which:
FIG. 1 is a block diagram illustrating a medical apparatus according to an embodiment of the present invention;
FIG. 2 is a block diagram illustrating an ultrasonic probe as an example of a capture unit of FIG. 1;
FIG. 3A is a front view of a slide bar as a type of hardware device according to an embodiment of the present invention;
FIG. 3B is a cross-sectional view of a hardware device illustrated in FIG. 3A;
FIG. 4 is a flowchart for describing a method of operating a medical apparatus according to an embodiment of the present invention;
FIGS. 5A and 5B are reference diagrams for describing a user interface using a hardware device according to an embodiment of the present invention;
FIGS. 6A to 6C are reference diagrams for describing a user interface using a hardware device according to another embodiment of the present invention; and
FIGS. 7A and 7B are reference diagrams for describing a user interface using a hardware device according to another embodiment of the present invention.

### DETAILED DESCRIPTION

Reference will now be made in detail to embodiments, examples of which are illustrated in the accompanying drawings, wherein like reference numerals refer to like elements throughout. In this regard, the present embodiments may have different forms and should not be construed as being limited to the descriptions set forth herein. Accordingly, the embodiments are merely described below, by referring to the figures, to explain aspects of the present description.

Hereinafter, embodiments of the present invention will be described in detail with reference to the accompanying drawings. Like reference numerals in the drawings denote like elements, and thus their descriptions will not be repeated.

The term "object" used herein may include a person, an animal, a part of the person or animal. For example, an object may include an organ such as a liver, a heart, a womb, a brain, breasts, an abdomen, or the like, or a blood vessel. Moreover, the term "user" used herein is a medical expert, and may be a doctor, a nurse, a medical technologist, a medical image expert, or the like, or may be an engineer who repairs a medical apparatus. However, the user is not limited thereto.

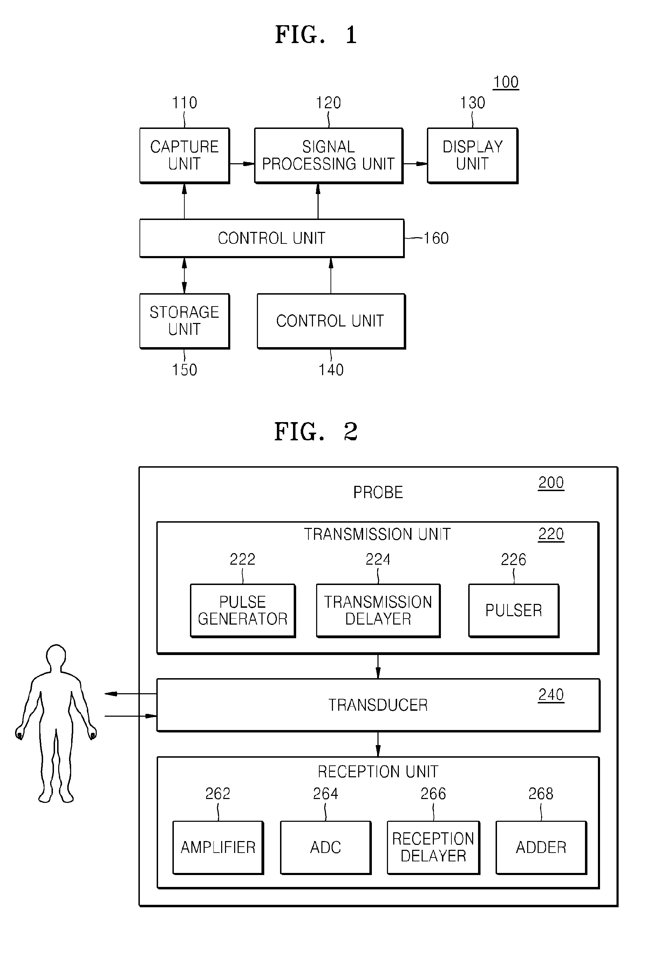

FIG. 1 is a block diagram illustrating a medical apparatus 100 according to an embodiment of the present invention. Referring to FIG. 1, the medical apparatus 100 includes a capture unit 110 that captures an object, a signal processing unit 120 that processes a signal applied from the capture unit 110 to generate an image, a display unit 130 that displays the image, a user input unit 140 that receives a user command, a storage unit 150 that stores various information, and a control unit 160 that controls an overall operation of the medical apparatus 100.

Elements of the capture unit 110 may be changed depending on a source that is used to capture an object. For example, when a source for capturing an object is an ultrasonic wave, the capture unit 110 may include a probe that transmits an ultrasonic wave to the object, and receives an ultrasonic echo signal reflected from the object. Alternatively, when a source for capturing an object is X-rays, the capture unit 110 may include an X-ray source, which generates X-rays, and an X-ray detector that detects the X-rays passing through the object. Hereinafter, an ultrasonic wave will be described as a source for capturing an object, but is not limited thereto. As another example, the medical apparatus 100 may generate an image by using X-rays or magnetic resonance.

FIG. 2 is a block diagram illustrating an ultrasonic probe 200 as an example of the capture unit 110 of FIG. 1. Referring to FIG. 2, the ultrasonic probe 200 is a device that may transmit an ultrasonic signal to an object and receive an echo signal reflected from the object to generate ultrasonic data, and may include a transmission unit 220, a transducer 240, and a reception unit 260.

The transmission unit 220 supplies a driving signal to the transducer 240. The transmission unit 220 may include a pulse generator 222, a transmission delayer 224, and a pulser 226.

The pulse generator 222 generates a rate pulse for generating a transmission ultrasonic wave based on a pulse repetition frequency (PRF). The transmission delayer 224 applies a delay time, used to decide a transmission directionality, to the rate pulse generated by the pulse generator 222. A plurality of the rate pulses with the delay time applied thereto correspond to a plurality of piezoelectric vibrators included in the transducer 240, respectively. The pulser 226 applies a driving signal (or a driving pulse) to the transducer 240 at a timing which corresponds to each of the plurality of rate pulses with the delay time applied thereto.

The transducer 240 transmits an ultrasonic wave to an object according to the driving signal supplied from the transmission unit 220, and receives an ultrasonic echo signal reflected from the object. The transducer 240 may include the plurality of piezoelectric vibrators that convert an electrical signal into sound energy (or vice versa).

The reception unit 260 processes a signal received from the transducer 240 to generate ultrasonic data, and may include an amplifier 262, an analog-to-digital converter (ADC) 264, a reception delayer 266, and an adder 268.

The amplifier 262 amplifies the signal received from the transducer 240, and the ADC 264 analog-to- digital converts the amplified signal. The reception delayer 266 applies a delay time, used to decide a reception directionality, to the digital-converted signal. The adder 268 adds signals processed by the reception delayer 266 to generate ultrasonic data. A reflection component from a direction defined on the reception directionality, may be emphasized by an addition processing performed by the adder 268.

The signal processing unit 120 processes data received from the capture unit 110 to generate an image. When the capture unit 110 is the ultrasonic probe 200, the signal processing unit 120 processes the ultrasonic data generated by the probe 200 to generate an ultrasonic image. The ultrasonic image may include at least one of a brightness (B) mode image in which a level of an ultrasonic echo signal reflected from an object is expressed as brightness, a Doppler mode image in which an image of a moving object is expressed as a spectrum type by using the Doppler effect, a motion (M) mode image that shows a motion of an object with time at a certain place, an elastic mode image in which a reaction difference between when compression is applied to an object and when compression is not applied to the object is expressed as an image, and a color (C) mode image in which a speed of a moving object is expressed as a color by using the Doppler effect. An ultrasonic image generating method uses a currently executable method, and thus, its detailed description is not provided. Therefore, an ultrasonic image according to an embodiment of the present invention may include at least one of mode-dimensional images such as a one-dimensional (1 D) image, a two-dimensional (2D) image, a three-dimensional (3D) image, and a four-dimensional (4D) image.

The display unit 130 displays information obtained through processing by the medical apparatus 100. For example, the display unit 130 may display an ultrasonic image generated by the signal processing unit 120, and display a graphics user interface (GUI) for requesting a user's input.

The display unit 130 may include at least one of a liquid crystal display (LCD), a thin film transistor-liquid crystal display (TFT-LCD), an organic light-emitting diode (OLED), a flexible display, a 3D display, and an electrophoretic display. Ultrasonic diagnosis apparatuses may include two or more display units 130 depending on an implementation type.

The user input unit 140 enables a user to input data for controlling the medical apparatus 100. The user input unit 140 may include a keypad, a mouse, a touch panel, a track ball, etc. The user input unit 140 is not limited to an illustrated configuration, and may further include various input devices such as a jog wheel, a jog switch, etc.

A touch panel may detect a real touch in which a pointer actually touches a screen, and moreover, may detect a proximity touch in which the pointer approaches a position which is separated from the screen by a certain distance. The pointer used herein denotes a touch instrument for actually touching or proximity-touching a specific portion of the touch panel. Examples of the pointer include an electronic pen, a finger, etc.

The touch panel may be implemented as a touch screen that configures the display unit 130 and a layer structure, and may be implemented as a contact capacitive type, a press resistive type, an infrared sensing type, a surface ultrasonic conductive type, an integration tension measurement type, and a piezo effect type. The touch screen performs a function of the user input unit 140 as well as the display unit 130, and thus is high in usability.

Although not shown, the touch panel may include various sensors, disposed inside or near the touch panel, for sensing a touch. An example of a sensor for sensing a touch of the touch panel is a tactile sensor. The tactile sensor denotes a sensor that senses a touch by a specific object by a degree, in which a user feels, or more. The tactile sensor may sense various pieces of information such as a roughness of a touched surface, a stiffness of a touched object, a temperature of a touched point, etc.

Another example of a sensor for sensing a touch of the touch panel is a proximity sensor. The proximity sensor denotes a sensor that detects an object approaching a detection surface or an object near the detection surface by using an electromagnetic force or infrared light without any mechanical contact. Examples of the proximity sensor include a transmissive photosensor, a directly reflective photosensor, a mirror reflective photosensor, a high frequency oscillation-type proximity sensor, a capacitive proximity sensor, a magnetic proximity sensor, and an infrared proximity sensor.

The storage unit 150 stores various information obtained through processing by the medical apparatus 100. For example, the storage unit 150 may store medical data, associated with a diagnosis of an object, such as an image. Also, the storage unit 150 may store an algorithm or a program which is executed in the medical apparatus 100. In particular, the storage unit 150 may store a lookup table in which identification information of a below-described hardware device is mapped to object information of when the hardware device operates as an object of a user interface.

The storage unit 150 may include at least one type of storage medium of a flash memory, a hard disk, a multimedia micro card, a card type memory (a secure digital (SD) card, an extreme digital (XD) card, or the like), a random access memory (RAM), a static random access memory (SRAM), a read-only memory (ROM), an electrically erasable programmable read-only memory (EEPROM), and a programmable read-only memory (PROM). Also, the medical apparatus 100 may operate by using web storage or a cloud server which performs a storage function of the storage unit 150 on the Web.

The control unit 160 controls an overall operation of the medical apparatus 100. That is, the control unit 160 may control an operation of each of the capture unit 110, signal processing unit 120, and display unit 130 of FIG. 1. For example, the control unit 160 may perform control in order for the signal processing unit 120 to generate an image, by using a user command input through the user input unit 140 or the program stored in the storage unit 150. Also, the control unit 160 may allow the display unit 130 to display the image generated by the signal processing unit 120.

Some or all of the signal processing unit 120, the user input unit 140, and the control unit 160 may be operated by a software module, but are not limited thereto. Also, some of the capture unit 110, the signal processing unit 120, the user input unit 140, and the control unit 160 may be operated by hardware. In addition, some functions of the control unit 160 may be respectively included in the capture unit 110, the signal processing unit 120, the user input unit 140, and the control unit 160, and their implementation type is not limited.

The touch screen performs a function of the user input unit 140 as well as the display unit 130, and due to this, the touch screen is high in usability. However, when the user input unit 140 is implemented as the touch screen, a user who uses the existing hardware device as the user input unit 140 may not be skilled in relation to the touch screen, and thus may experience inconvenience. Also, even when a user uses the touch screen, the use may desire to maintain a physical sense when inputting a user command. The following description is of a method that mounts a hardware device on a partial area of the touch screen, and uses the hardware device as a user input device.

The hardware device denotes an device in which when the hardware device is mounted on the touch screen, the hardware device is recognized as an object of a user interface, and a user inputs a user command by moving a partial area of the hardware device. Since the medical apparatus 100 is used in a space requiring thorough hygiene, the hardware device may be a disposable product. Also, the hardware device may be formed of a cleanable material, and may be antibiotic-coated.

Here, the object of the user interface is for inputting a user command to the medical apparatus 100, and may include an icon, a text, or an image. In the embodiment, the hardware device may become the object of the user interface. For example, the hardware device may include a trackball, a knob, a button, or a slide bar.

As the object of the user interface, the hardware device may include an identification unit that identifies the hardware device as the object of the user interface and an operation unit that transfers a user command to the touch screen though a motion. Also, the hardware device may include a housing that forms an external appearance of the hardware device and supports the identification unit and the operation unit.

The identification unit may be provided on a surface of the housing facing the touch screen when the hardware device is mounted on the touch screen. Therefore, when the hardware device is mounted on the touch screen, the touch screen may recognize the identification unit. The identification unit is for identifying what object the hardware device is in the user interface, and may be a pattern representing the external appearance of the hardware device. That is, the identification unit may be configured with a shape and size of the pattern. For example, when the hardware device operates as the slide bar having a tetragonal shape, the identification unit may be a pattern having the same tetragonal shape as the external appearance of the slide bar, and the identification unit may be disposed on a surface of the housing facing the touch screen. Therefore, when the hardware device is mounted on the touch screen, the touch screen may sense a tetragonal pattern, and the medical apparatus 100 may recognize the hardware device as the object of the user interface which is the slide bar.

The operation unit may be disposed on an externally exposed surface of the housing when the hardware device is mounted on the touch screen. When the hardware device has been mounted on the touch screen, the identification unit and housing of the hardware device are fixed. However, the operation unit may be moved by a pressure or the like to touch the touch screen. Therefore, a user may input a user command through the operation unit. For example, when the hardware device is an input apparatus that is operable as a keyboard, the operation unit may include various functional keys of the keyboard.

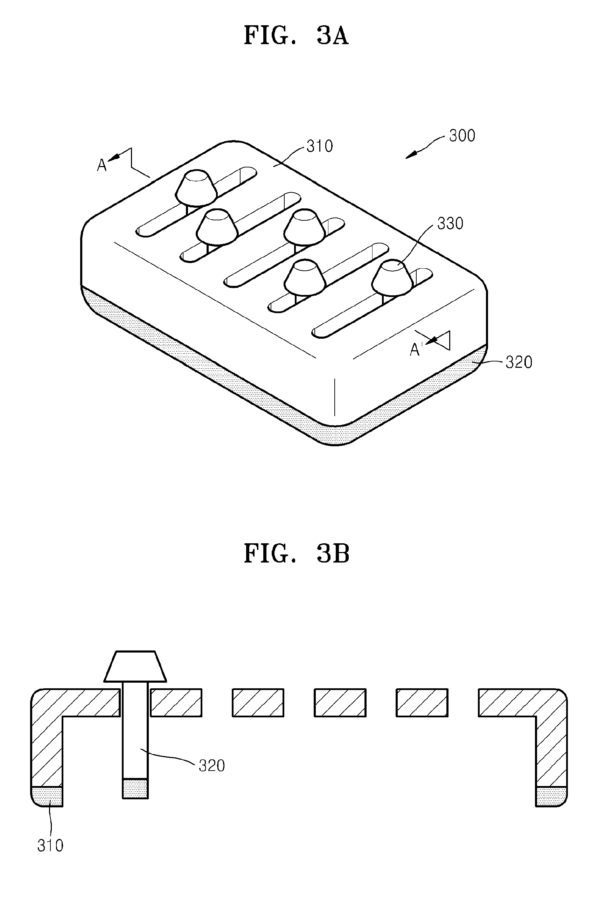

FIG. 3A is a front view of a slide bar as a type of hardware device according to an embodiment of the present invention, and FIG. 3B is a cross-sectional view of a hardware device illustrated in FIG. 3A. As illustrated in FIGS. 3A and 3B, a hardware device 300 may include a housing 310 that forms an external appearance of a slide bar, an identification unit 320 that is configured with a pattern which is formed at an externally exposed surface of the housing 310 facing the touch screen, and an operation unit 330 that is disposed at an externally exposed surface of the housing 310. The identification unit 320 may be configured with a pattern for identifying an overall shape and size of the hardware device 300. Therefore, when the hardware device 300 is mounted on the touch screen, the touch screen may sense the pattern, and the control unit 160 of the medical apparatus 100 may recognize the hardware device 300 as the object of the user interface that is the slide bar. Also, a user may move the operation unit 330. At this time, the touch screen may sense a motion of the operation unit 330, and the control unit 160 of the medical apparatus 100 may recognize what user command is input, by using the motion of the operation unit 330. As described above, the hardware device 300 may be the object of the user interface which is configured in various types, but a detailed description of kinds of hardware devicees will not be provided here.

Hereinafter, the medical apparatus 100 that recognizes the hardware device, which is mounted on the touch screen, as an input apparatus to operate, will be described.

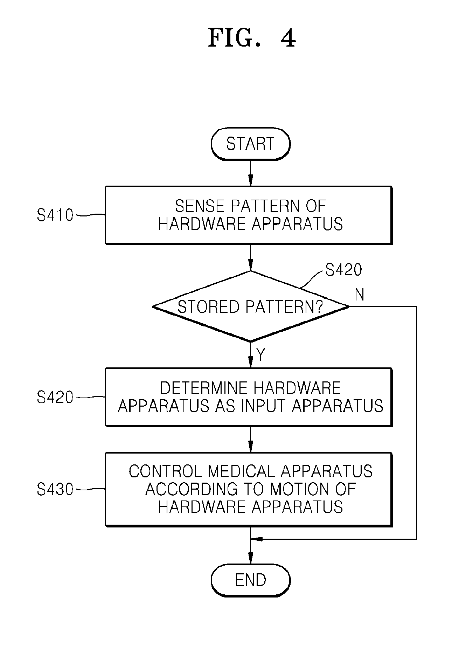

FIG. 4 is a flowchart for describing a method of operating a medical apparatus according to an embodiment of the present invention. Referring to FIG. 4, when the hardware device is mounted on the touch screen, the touch screen senses a pattern of the hardware device in operation S410. The hardware device may be mounted on the touch screen in order for the touch screen to sense the identification unit of the hardware device. When the pattern is formed at a surface of the hardware device facing the touch screen and the hardware device is mounted on the touch screen, the touch screen senses the pattern to transfer the sensed result to the control unit 160.

When the pattern of the hardware device matches a pattern stored in the storage unit 150 in operation S420-Y, the control unit 160 determines the hardware device as an input apparatus enabling a user command to be input in operation S430. The storage unit 150 stores the lookup table in which the pattern is mapped to the object of the user interface. Therefore, the control unit 160 determines whether the pattern received from the touch screen is included in the pattern stored in the storage unit 150, by using the lookup table. When the pattern of the hardware device matches the patterned stored in the storage unit 150, the control unit 160 may determine the hardware device as the object of the user interface matching the sensed pattern. That is, the control unit 160 determines the hardware device as the input apparatus enabling the user command to be input.

Then, the control unit 160 recognizes a motion of the hardware device as the user command to control the medical apparatus 100 in operation S440. A user may move the operation unit of the hardware device, and the touch screen senses the motion of the operation unit to transfer the sensed result to the control unit 160. The control unit 160 may recognize the motion of the operation unit as the user command to control the medical apparatus 100 according to the user command.

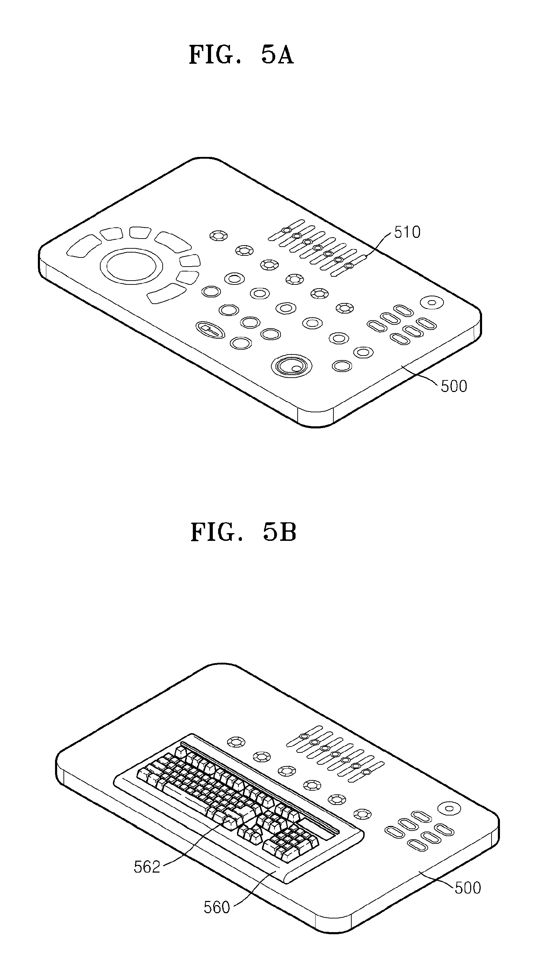

Next, a method in which the hardware device operates as the object of the user interface will be described in more detail with reference to the drawings. FIGS. 5A and 5B are reference diagrams for describing a user interface using a hardware device according to an embodiment of the present invention.

As illustrated in FIG. 5A, a plurality of objects 510 may be displayed on a touch screen 500. Here, each of the plurality of objects 510 may be a type of user interface, and a user may touch at least one of the plurality of objects 510 to input a user command.

The user, as illustrated in FIG. 5B, may mount a hardware device 560 on the touch screen 500. A pattern may be formed at a bottom of the hardware device 560, and the touch screen 500 senses the pattern to transfer the sensed result to the control unit 160. The control unit 160 checks whether the pattern is a pattern for a keyboard by using the lookup table stored in the storage unit 150, and determines the hardware device 560 as the keyboard that is a type of user interface. Furthermore, the control unit 160 recognizes an area of the touch screen 500, on which the keyboard is mounted, as a touch input space using the keyboard. The user may move at least one of a plurality of functional keys 562 included in the keyboard to input a user command, and the touch screen 500 may sense a motion of the moved functional key 562 to transfer the sensed result to the control unit 160. Thus, the control unit 160 may control the medical apparatus 100 according to the sensed motion of the moved functional key 562.

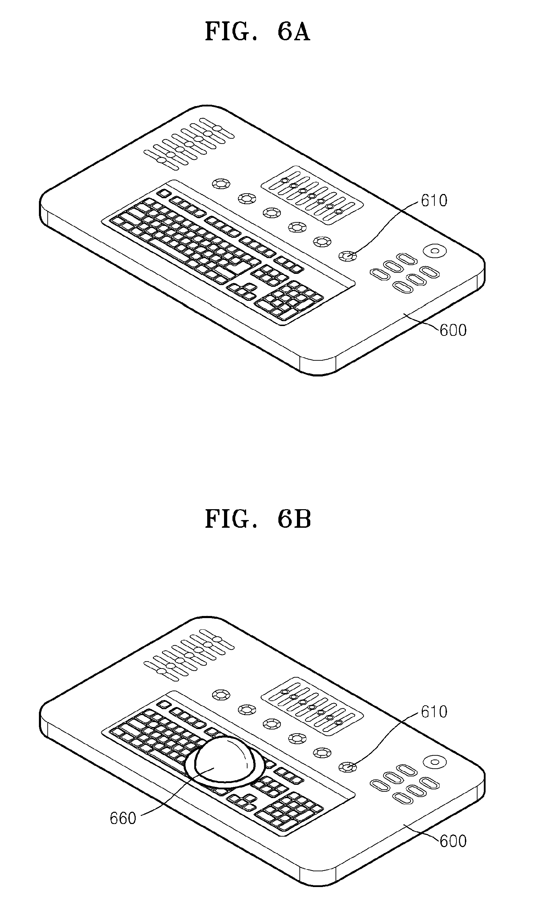

FIGS. 6A to 6C are reference diagrams for describing a user interface using a hardware device according to another embodiment of the present invention.

As illustrated in FIG. 6A, a plurality of objects 610 may be displayed on a touch screen 600. Here, each of the plurality of objects 610 may be a type of user interface, and a user may touch at least one of the plurality of objects 610 to input a user command.

As illustrated in FIG. 6B, a hardware device 660 may be mounted on the touch screen 600. Here, the hardware device 660 may be a trackball. The hardware device 660 may be mounted on the touch screen 600 such that an identification unit of the hardware device 660 faces a touch screen 600. Therefore, the touch screen 600 senses the identification unit, namely, a pattern, and transfers the sensed result to the control unit 160. The control unit 160 may determine the hardware device 660 having the sensed pattern as the trackball by using the lookup table. Furthermore, the touch screen 600 senses a motion in an area with the hardware device 660 mounted thereon, and the control unit 160 may recognize the sensed motion as a user command to control the medical apparatus 100.

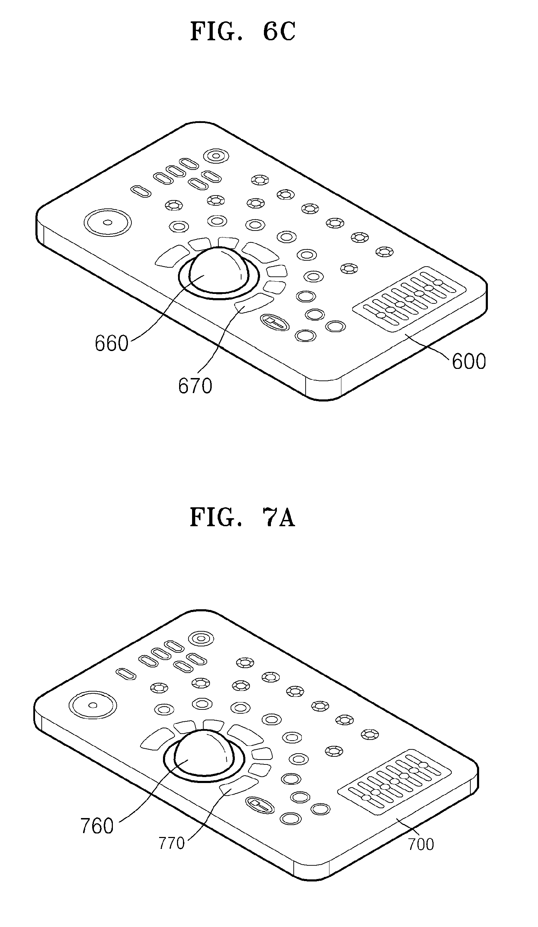

Moreover, as illustrated in FIG. 6C, the control unit 160 may further display an object 670 of a user interface associated with the hardware device 660, near an area of the touch screen 600 on which the hardware device 660 is mounted. For example, when the hardware device 660 that is the trackball operates as the object of the user interface for moving a position of a cursor displayed in the display unit 130, the control unit 160 may further display the object 670 (for example, an OK key), which enables selection of an item or a value displayed in an area with the cursor placed therein, in an area near an area of the touch screen 600 in which the hardware device 660 is displayed.

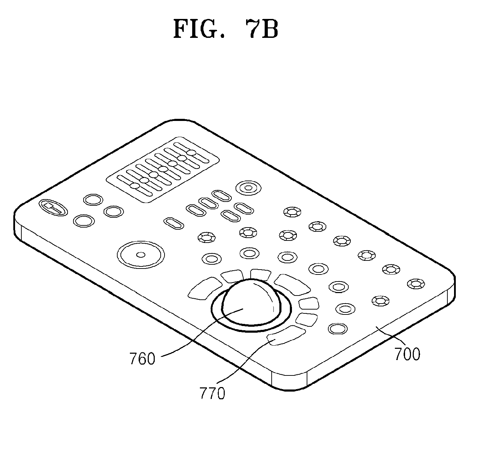

FIGS. 7A and 7B are reference diagrams for describing a user interface using a hardware device according to another embodiment of the present invention.

As illustrated in FIG. 7A, a hardware device 760 is disposed in an area of a touch screen 700. The control unit 160 displays an object 770 of a user interface associated with the hardware device 760, near the hardware device 760.

A user may change a position of the hardware device 760 on the touch screen 700. For example, the user may separate the hardware device 700 from the touch screen 700, and dispose the hardware device 760 in another area of the touch screen 700. Alternatively, with the hardware device 760 being disposed on the touch screen 700, the hardware device 760 may be dragged to thereby be moved. Then, as illustrated in FIG. 7B, the control unit 160 may determine a position of the hardware device 760 as being changed, and an object of a user interface associated with the hardware device 760 may be moved and displayed with respect to the changed position.

The user interface using the hardware device is not limited to the medical apparatus 100. The user interface may be applied to electronic devices such as image devices usable as a user input unit.

Moreover, in the embodiment, the control unit is described as the control unit of the medical apparatus, but is not limited thereto. A separate control unit may be provided in the user input unit. The separate control unit in the user input unit may identify a hardware device, determine a motion of the hardware device as a user command, and transfer the determined result to the control unit of the medical apparatus.

Since a hardware device is usable as an object of a user interface, a user experiences a physical sense by using the hardware device even when the user input unit is implemented as the touch screen. Also, the medical apparatus displays an object of another user interface depending on a kind of hardware device, and thus, the user operates the medical apparatus more conveniently.

It should be understood that the exemplary embodiments described herein should be considered in a descriptive sense only and not for purposes of limitation. Descriptions of features or aspects within each embodiment should typically be considered as available for other similar features or aspects in other embodiments.

While one or more embodiments of the present invention have been described with reference to the figures, it will be understood by those of ordinary skill in the art that various changes in form and details may be made therein without departing from the spirit and scope of the present invention as defined by the following claims.

## Claims

1. A method of operating a medical apparatus, the method comprising:
sensing a pattern of a hardware device disposed on a touch screen; and
determining the hardware device as an input apparatus enabling a user command to be input when the sensed pattern matches a stored pattern.

2. The method of claim 1, wherein the pattern of the hardware device is formed at a surface of the hardware device facing the touch screen

3. The method of claim 1 or 2, further comprising controlling the medical apparatus according to a motion of the hardware device.

4. The method of claim 3, wherein the motion of the hardware device corresponds to a touch that is sensed in an area of the touch screen in which the hardware device is disposed, or, wherein the motion of the hardware device is a motion of a partial area of the hardware device when a housing for the hardware device is fixed on the touch screen.

5. The method of any one of claims 1 to 4, further comprising displaying an object of a user interface associated with the hardware device, in an area near an area of the touch screen in which the hardware device is disposed.

6. The method of claim 5, wherein when a position of the hardware device disposed on the touch screen is changed, a position of the object of the user interface is changed, and the changed object is displayed.

7. The method of any one of claims 1 to 6, wherein the hardware device comprises at least one of a trackball, a knob, a button, a slide bar, and a keyboard.

8. The method of any one of claims 1 to 7, wherein the medical apparatus is a disposable apparatus.

9. A medical apparatus comprising:
a touch screen that senses a pattern of a hardware device;
a storage unit that stores a pattern and an object of a user interface, the pattern being mapped to the object of the user interface; and
a control unit that, when a pattern of a hardware device disposed on the touch screen is included in the pattern stored in the storage unit, determines the hardware device as an input apparatus enabling a user command to be input.

10. The medical apparatus of claim 9, wherein the pattern of the hardware device is formed at a surface of the hardware device facing the touch screen.

11. The medical apparatus of claim 9 or 10, wherein the control unit controls the medical apparatus according to a motion of the hardware device.

12. The medical apparatus of claim 11, wherein the motion of the hardware device corresponds to a touch that is sensed in an area of the touch screen in which the hardware device is disposed, or, wherein the motion of the hardware device is a motion of a partial area of the hardware device when a housing for the hardware device is fixed on the touch screen.

13. The medical apparatus of any one of claims 9 to 12, wherein the control unit further displays an object of a user interface associated with the hardware device, in an area near an area of the touch screen in which the hardware device is disposed.

14. The method of claim 13, wherein when a position of the hardware device disposed on the touch screen is changed, the control unit changes a position of the object of the user interface and displays the changed object.

15. The method of any one of claims 9 to 14, wherein the hardware device comprises at least one of a trackball, a knob, a button, a slide bar, and a keyboard.
